(19) Europäisches Patentamt
European Patent Office
Office européen des brevets

(11) Numéro de publication : 0 052 115 B1

(12) # FASCICULE DE BREVET EUROPÉEN

(45) Date de publication du fascicule du brevet : 07.12.83

(51) Int. Cl.³ : C 07 C 49/557, C 07 C 45/45, C 11 B 9/00

(21) Numéro de dépôt : 81901148.7

(22) Date de dépôt : 12.05.81

(86) Numéro de dépôt international : PCT/CH 81/00052

(87) Numéro de publication internationale : WO WO/81033 (26.11.81 Gazettee 81/28)

(54) **COMPOSES BICYCLIQUES ET LEUR UTILISATION EN TANT QU'AGENTS PARFUMANTS.**

(30) Priorité : 22.05.80 CH 4008/80

(43) Date de publication de la demande : 26.05.82 Bulletin 82/21

(45) Mention de la délivrance du brevet : 07.12.83 Bulletin 83/49

(84) Etats contractants désignés : CH DE FR GB LI NL

(56) Documents cités :
FR-A- 2 259 091
FR-A- 2 425 420
US-A- 4 143 074

(73) Titulaire : FIRMENICH SA
Case postale 239
CH-1211 Genève 8 (CH)

(72) Inventeur : NÄF, Ferdinand
11, ch. des Crêts de Champel
CH-1206 Genève (CH)
Inventeur : DECORZANT, René
3, av. du Gros-chêne
CH-1213 Onex/GE (CH)
Inventeur : SCHULTE-ELTE, Karl-Heinrich
44, ch. de Carabot
CH-1213 Onex/GE (CH)

(74) Mandataire : Salvadori, Giuseppe, Dr.
c/o Firmenich S.A. Case Postale 239
CH-1211 Genève 8 (CH)



Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

Composés bicycliques et leur utilisation en tant qu'agents parfumants

## Domaine technique

La présente invention a trait au domaine de la parfumerie, elle concerne plus particulièrement les composés de formule

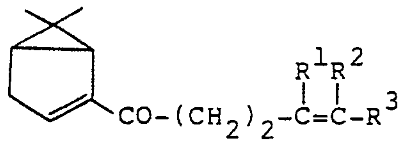

(I)

dans laquelle chacun des symboles $R^1$, $R^2$ et $R^3$, identiques ou différents, représente un atome d'hydrogène ou un reste alkyle inférieur contenant 1 à 3 atomes de carbone, de préférence un groupe méthyle. L'invention concerne en outre l'utilisation desdits composés de formule (I) en tant qu'agents parfumants pour la préparation de parfums et produits parfumés.

## Etat de la technique

L'art antérieur fait état de certains composés ayant une structure similaire.

La demande française FR-A-2 425 420, dans sa revendication principale, décrit des composés de formule

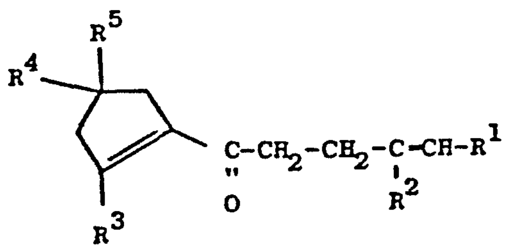

dans laquelle chacun des symboles $R^1$, $R^2$, $R^3$ et $R^4$ représente un atome d'hydrogène ou un groupe méthyle et $R^5$ représente un radical alkyle, linéaire ou ramifié, contenant de 1 à 3 atomes de carbone, et, dans la revendication 4, décrit leur utilisation à titre d'ingrédients pour la préparation de parfums et de produits parfumés.

Le brevet US-A-4 143 074 décrit une série de dérivés du norbornane parmi lesquels figurent les dérivés particuliers que voici :

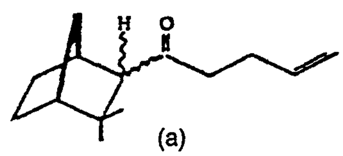

(a)

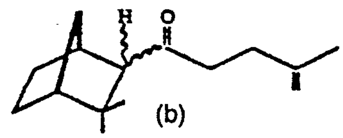

(b)

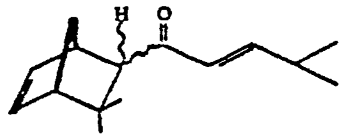

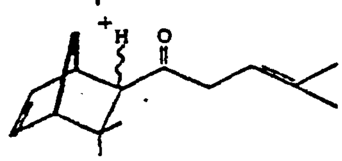

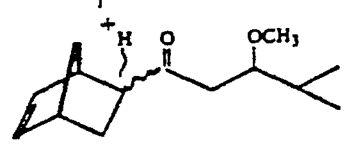

(c)

D'une manière générale, les dérivés du norbornane décrits possèdent des caractères odorants très variés définis comme étant entre autres (voir colonne 2, lignes 22 à 29) :

intenses et plaisants, doux, boisés, fruités, épicés et herbacés.

De façon plus spécifique,

— le composé (a) possède une note douce, fruitée, balsamique, florale avec un caractère boisé et pamplemousse,

— le composé (b), qui est constitué par ailleurs d'un mélange de deux composants, possède une note boisée, fruitée,

— le composé (c), qui est également constitué par un mélange, possède une note fruitée de type baies.

La demande française FR-A-2 259 091 décrit des composés de formule

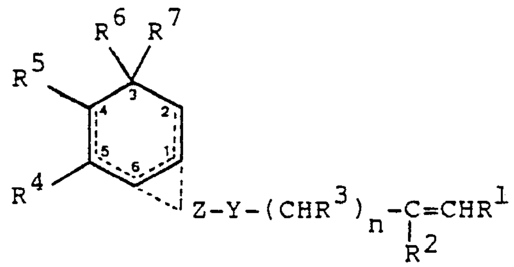

possédant un cycle à six membres saturé, ou possédant une double liaison en position 1, 4 ou 6, ou encore deux doubles liaisons en positions 1 et 4, 1 et 5, ou 4 et 6 du cycle comme indiqué par les pointillés, et dans laquelle

— l'indice n vaut 0, 1 ou 2 ;

— le symbole Z, fixé en position 1 ou 6 comme indiqué par les pointillés, représente un groupe CO, $COR^8$ ou $C(OR^9)_2$ dans lesquels le symbole $R^8$ représente un reste acyle contenant de 1 à 6 atomes de carbone et $R^9$ représente un reste alkyle ou alkylène contenant de 1, respectivement 2, à 6 atomes de carbone ;

— le symbole Y représente un atome d'oxygène ou un groupe $CH_2$ ;

— chacun des symboles $R^1$, $R^2$, $R^3$, $R^4$ et $R^5$ représente un atome d'hydrogène ou l'un d'entre eux représente un groupe $CH_3$ et les autres un atome d'hydrogène, et chacun des symboles $R^6$ et $R^7$ représente un reste alkyle contenant de 1 à 3 atomes de carbone ou l'un d'eux un reste alkyle défini comme ci-dessus et l'autre un atome d'hydrogène, ainsi que leur utilisation à titre d'ingrédients parfumants. Lesdits composés sont caractérisés par une odeur « puissante dont la note verte, herbacée rappelle notamment celle de l'essence ou du résinoïde de galbanum ». Ils ont en outre pour effet de « renforcer ou développer, au sein des compositions auxquelles ils sont incorporés, une note verte, montante, d'une grande distinction ».

## Exposé de l'invention

Les composés bicycliques cétoniques de formule (I) sont des composés nouveaux que l'on peut synthétiser à l'aide d'un procédé original à partir de $\Delta^3$-carène comme illustré par le schéma que voici :

Schéma

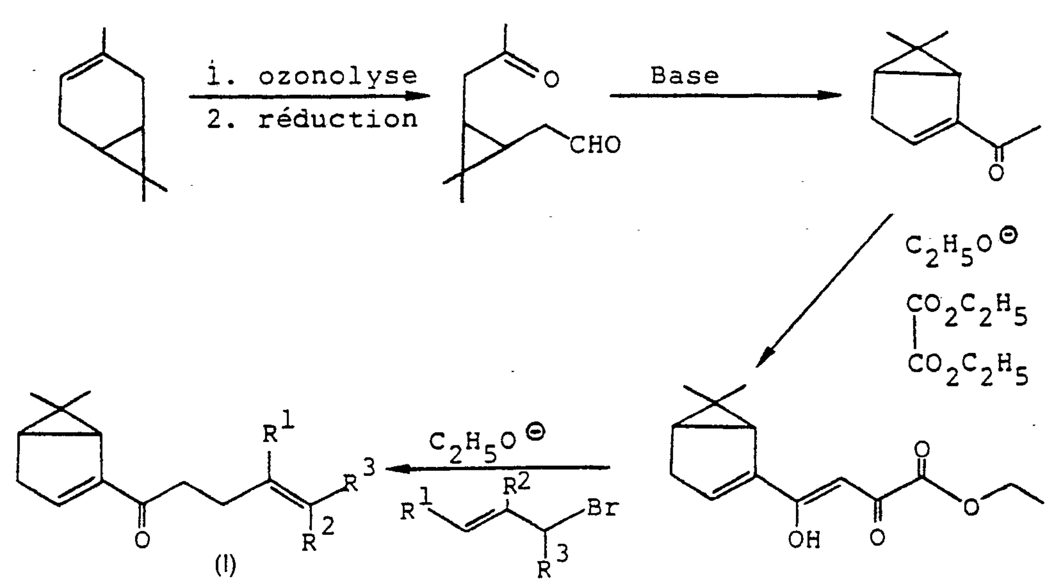


(I)

Les deux premières étapes du procédé décrit ci-dessus peuvent être effectuées conformément à des méthodes connues [voir par exemple le brevet britannique N° 1'435'887, p. 12] tandis que la transformation du 2-acétyl-6,6-diméthyl-bicyclo [3.1.0] hex-2-ène en leurs dérivés de formule (I) est illustrée en détail dans les exemples de préparation qui suivent. Dans le domaine de la parfumerie, les composés de formule (I) se caractérisent par leur odeur originale de type frais et vert. Leur caractère odorant rappelle la note verte de l'essence de Galbanum tout en étant très riche et surtout très fleurie et puissante.

De par leurs propriétés, les composés de l'invention trouvent une utilisation fort étendue dans la manufacture de compositions parfumantes de nature variée ; en outre ils s'harmonisent convenablement avec les ingrédients d'emploi courant en parfumerie, leur utilisation est donc générale, tant en parfumerie fine que pour le parfumage d'articles tels que savons, détergents, produits d'entretien ou cosmétiques.

Les quantités des composés de formule (I) à utiliser afin d'obtenir des effets parfumants intéressants varient dans une gamme de valeur assez étendue et dépendent de la nature des coingrédients dans une composition parfumante donnée ou de l'effet recherché. Des quantités de l'ordre de 0,5 % du composé actif en poids, par rapport au poids total de la composition dans laquelle ils sont incorporés, servent déjà à conférer un effet marqué. Bien entendu, des proportions inférieures, par exemple d'environ 0,1 ou 0,2 %, peuvent être employées lors de la préparation d'articles parfumés, lesdites valeurs ne représentant cependant pas des limites absolues.

Parmi les composés bicycliques de l'invention il convient tout particulièrement de mentionner les suivants :

2-[pent-4-ène-1-oyle]-6,6-diméthyl-bicyclo [3.1.0] hex-2-ène,
2-[hex-4-ène-1-oyle]-6,6-diméthyl-bicyclo [3.1.0] hex-2-ène,
2-[4-méthyl-pent-4-ène-1-oyle]-6,6-diméthyl-bicyclo [3.1.0] hex-2-ène, et
2-[5-méthyl-hex-4-ène-1-oyle]-6,6-diméthyl-bicyclo [3.1.0] hex-2-ène.

L'invention sera illustrée de façon plus détaillée par les exemples ci-après. Dans lesdits exemples, les températures sont indiquées en degrés centigrades et les abréviations ont le sens usuel dans l'art.

Exemple 1

Préparation de 2-[pent-4-ène-1-oyle]-6,6-diméthyl-bicyclo [3.1.0] hex-2-ène

a) un mélange de 15 g (0,1 M) de 2-acétyl-6,6-diméthyl-bicyclo [3.1.0] hex-2-ène et 21,9 g (0,15 M) d'oxalate de diéthyle a été ajouté à − 10/− 15° à une solution de 2,76 g (0,12 M) de sodium dans 50 ml d'éthanol anhydre. Le mélange de réaction a été maintenu sous agitation pendant environ 2 heures à température ambiante puis il a été neutralisé avec une solution aqueuse à 10 % d'HCl. Par extraction à l'éther diéthylique et séparation des phases organiques suivie de séchage et évaporation on a obtenu 27,3 g d'un résidu qui par distillation a fourni 11,7 g de 6,6-diméthyl-2-[2-hydroxy-3-oxo-3-carbéthoxy-prop-1-ène-1-yl]-bicyclo [3.1.0] hex-2-ène ayant Eb. 140°/0,5 Torr.

b) 7,73 g (0,031 M) du produit obtenu sous lettre a) ont été ajoutés à température ambiante à une solution de 0,86 g (0,037 M) de sodium dans 30 ml d'éthanol anhydre et la solution résultante a été maintenue sous agitation à 25-30° pendant 10 minutes. Une solution de 3,7 g (0,031 M) de bromure d'allyle dans 15 ml d'éthanol a été ensuite ajoutée au mélange obtenu et le tout a été agité pendant 20 h à 75°.

Après évaporation des parties volatiles, adjonction d'eau et extraction avec de l'éther diéthylique, suivie des traitements usuels, on a obtenu 6,45 g d'un résidu, qui par distillation fractionnée, a fourni une fraction ayant Eb. 115-120°/10 Torr. Par purification de cette fraction au moyen de chromatographie sur colonne remplie avec du $SiO_2$ on a obtenu 3,33 g (rend. 56,7 %) du produit désiré (éluant : hexane/éther diéthylique : 98/2) dont les caractères analytiques étaient les suivants :

SM : m/e = 190 (14), 175 (61), 162 (14), 148 (24), 135 (100), 119 (44), 107 (52), 91 (78), 79 (34), 65 (43), 55 (68), 43 (35), 29 (26) ;

RMN (60 MHz, $CDCl_3$) : 0,75 (3H, s) ; 1,10 (3H, s) ; 1,39 (1H, dxd, $J_1 = J_2 = 7Hz$) ; 2,00-2,89 (7H, m) ; 4,96 (1H, d, J = 11Hz) ; 5,02 (1H, d, J = 17Hz) ; 5,55-6,10 (1H, m) ; 6,50 (1H, t) δ ppm.

Le 2-acétyl-6,6-diméthyl-bicyclo [3.1.0] hex-2-ène, utilisé comme produit de départ dans le procédé décrit ci-dessus, peut être obtenu conformément à la méthode décrite dans le brevet britannique N° 1'435'887 ou selon Agric. Biol. Chem. *31*, 33 (1967).

Les autres composés de formule (I) suivant l'invention peuvent être obtenus par un procédé similaire, le bromure d'allyle étant remplacé par le bromure d'alcényle correspondant.

Les produits ainsi préparés montraient les caractères analytiques suivants :

2-[hex-4-ène-1-oyle]-6,6-diméthyl-bicyclo [3.1.0] hex-2-ène

RMN (60 MHz) : 0,75 (3H, s) ; 1,10 (3H, s) ; 1,37 (1H, dxd, $J_1 = 7Hz$ ; $J_2 = 7Hz$) ; 1,67 (3H, m) ; 2,00-2,86 (7H, m) ; 5,38-5,55 (2H, m) ; 6,49 (1H, m) δ ppm ;

SM : $M^+ = 204$ (39) ; m/e : 189 (65), 175 (32), 161 (24), 149 (14), 135 (100), 119 (42), 107 (61), 93 (74), 91 (79), 85 (43), 65 (50), 55 (63), 41 (64), 29 (45).

2-[4-méthyl-pent-4-ène-1-oyle]-6,6-diméthyl-bicyclo [3.1.0] hex-2-ène

RMN (90 MHz) : 0,76 (3H, s) ; 1,11 (3H, s) ; 1,38 (1H, dxd, $J_1$ = 7Hz, $J_2$ = 7Hz) ; 1,74 (3H, s) ; 2,04-2,86 (7H, m) ; 4,66 (1H, s) ; 4,70 (1H, s) ; 6,48 (1H, s) δ ppm ;
SM : $M^+$ = 204 (10) ; m/e : 189 (30), 176 (10), 162 (17), 147 (10), 135 (100), 119 (34), 107 (43), 93 (60), 91 (74), 79 (32), 65 (45), 55 (31), 41 (77).

2-[5-méthyl-hex-4-ène-1-oyle]-6,6-diméthyl-bicyclo [3.1.0] hex-2-ène

RMN (90 MHz) : 0,76 (3H, s) ; 1,11 (3H, s) ; 1,38 (1H, dxd, $J_1$ = 7Hz, $J_2$ = 7Hz) ; 1,64 (3H, s) ; 1,69 (3H, s) ; 2,04-2,86 (7H, m) ; 5,09 (3H, t, J = 7Hz) ; 6,45 (1H, s) δ ppm ;
SM : $M^+$ = 218 (39) ; m/e : 203 (19), 189 (1), 175 (30), 161 (3), 150 (37), 135 (55), 121 (11), 107 (45), 91 (56), 79 (24), 69 (65), 55 (32), 41 (100).

## Exemple 2

Parfumage de savon

On a procédé au parfumage d'une pâte de savon commerciale en y ajoutant 0,1 % en poids du composé de formule (I) ($R_1 = R_2 = R_3$ = H), et, à l'aide de la pâte ainsi parfumée, on a procédé à la préparation de savon de toilette suivant la méthode usuelle. Les produits ainsi obtenus possédaient une note olfactive verte et plaisante.

## Exemple 3

Parfumage d'une poudre détergente

Une poudre détergente du commerce à odeur neutre a été parfumée à l'aide du composé de formule (I) ($R_1 = R_2 = R_3$ = H) à raison de 0,05 % en poids. Le produit résultant possédait une odeur agréable de type vert-fleuri.

## Exemple 4

Eau-de-toilette

En ajoutant à un échantillon d'eau-de-toilette classique 0,1 % du composé de formule (I) ($R_1 = R_2 = R_3$ = H), on a obtenu une composition présentant une odeur montante et un caractère floral enrichi.

## Exemple 5

Composition parfumante pour shampooings

On a préparé une composition parfumante pour shampoings en mélangeant les ingrédients suivants (parties en poids) :

| | |
|---|---|
| Salicylate de benzyle | 200 |
| Alcool phényl-éthylique | 100 |
| Acétate de benzyle | 80 |
| Acétate d'isononyle | 80 |
| Essence de Bois de rose du Brésil | 60 |
| Héliotropine | 50 |
| Hydroxycitronellal | 50 |
| Acétate de géranyle | 40 |
| Géraniol | 40 |
| Undécénal à 10 %* | 40 |
| Aldéhyde amylcinnamique | 30 |
| 1,1-Diméthyl-4-acétyl-6-tert-butylindane | 30 |
| α-Isométhylionone | 30 |
| DORINONE® α à 10 %*[1)] | 20 |
| Acétate de styrallyle | 20 |
| Ethyl-cyclopentylidène-acétate à 1 %* | 20 |
| Décanal à 10 %* | 20 |
| Salicylate d'amyle | 20 |
| Cyclosal | 20 |

| | |
|---|---|
| HEDIONE® 1) | 20 |
| DORICENONE® à 1 %* 1) | 10 |
| Aldéhyde anisique | 5 |
| MAYOL® 1) | 5 |
| TOTAL | 990 |

En ajoutant à cette composition de type « fleuri », 10 g (= 1 %) du composé de formule (I) ($R_1 = R_2 = R_3 = H$), on obtient une nouvelle composition dont le caractère odorant, plus montant et plus riche, présente une nuance verte de type galbanum.

Par l'utilisation, conformément aux Exemples 2 à 5, de l'un des composés homologues de formule (I) mentionnés à l'Exemple 1, on a observé des effets anologues. Ceux-ci étaient cependant moins prononcés dans le cas de l'utilisation du 2-[5-méthyl-hex-4-ène-1-oyle]-6,6-diméthyl-bicyclo [3.1.0] hex-2-ène.

**Revendications**

1. Un composé de formule (I)

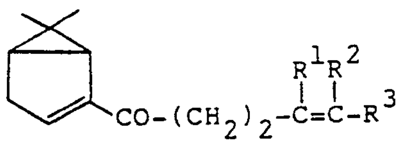

(I)

dans laquelle chacun des symboles $R^1$, $R^2$ et $R^3$, identiques ou différents, représente un atome d'hydrogène ou un reste alkyle inférieur contenant 1 à 3 atomes de carbone, de préférence un groupe méthyle.

2. Utilisation d'au moins un des composés de formule (I) suivant la revendication 1, en tant qu'agent parfumant pour la préparation de parfums et produits parfumés.

3. Composition parfumante caractérisée en ce qu'elle contient en tant qu'ingrédient actif au moins un des composés de formule (I) suivant la revendication 1.

4. Un produit parfumé résultant de l'utilisation suivant la revendication 2.

**Claims**

1. A compound of formula

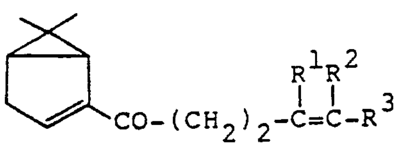

(I)

wherein each of symbols $R^1$, $R^2$ and $R^3$, identical or different, represents a hydrogen atom or a lower alkyl radical having 1 to 3 carbon atoms, preferably a methyl radical.

2. Utilization of at least one of the compounds of formula (I) according to claim 1, as a perfuming agent for the preparation of perfumes and perfumed products.

3. Perfume composition characterized in that it contains as active ingredient at least one of the compounds of formula (I) according to claim 1.

4. A perfumed product resulting from the utilization according to claim 2.

**Ansprüche**

1. Eine Verbindung der Formel

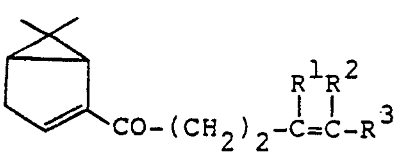

(I)

* dans le phtalate diéthylique

1) origine : FIRMENICH SA, Genève

worin jedes der Symbole $R^1$, $R^2$ und $R^3$, welche gleich oder verschieden sein können, ein Wasserstoffatom oder einen niederen Alkylrest mit 1 bis 3 C-Atomen, vorzugsweise eine Methylgruppe, darstellt.

2. Verwendung von mindestens einer der Verbindungen der Formel (I) gemäss Anspruch 1, als Geruchsbestandteil zur Herstellung von Parfümen und parfümierten Produkten.

3. Eine Parfümkomposition, dadurch gekennzeichnet, dass sie als aktiven Bestandteil mindestens eine der Verbindungen der Formel (I) gemäss Anspruch 1 enthält.

4. Ein parfümiertes Produkt, welches das Resultat der Verwendung gemäss Anspruch 2 ist.